# EUROPÄISCHE PATENTSCHRIFT

(11) **EP 3 086 746 B1**
(45) Veröffentlichungstag und Bekanntmachung des Hinweises auf die Patenterteilung: **12.09.2018**
(21) Anmeldenummer: 14825297.6
(22) Anmeldetag: 18.12.2014
(51) Int. Cl.: A61F 13/06, A61F 5/01, A61F 5/30

(54) **PATELLARSEHNENBANDAGE**
PATELLAR TENDON BANDAGE
BANDE DE MAINTIEN ROTULIEN

(30) Priorität: 23.12.2013 DE 102013022088
(43) Veröffentlichungstag der Anmeldung: 02.11.2016
(73) Patentinhaber: Bauerfeind AG, 07937 Zeulenroda (DE)
(72) Erfinder: SCHEUERMANN, Rainer, 24223 Raisdorf (DE); BÖCKELMANN, Joachim, 47906 Kempen (DE); BAUERFEIND, Hans B., 07937 Zeulenroda-Triebes (DE)
(74) Vertreter: Wohlfahrt, Jan Günther
(86) Internationale Anmeldenummer: PCT/EP2014/078380
(87) Internationale Veröffentlichungsnummer: WO 2015/097051

(56) Entgegenhaltungen:
- DE-U1- 29 803 103
- US-A- 4 287 884
- US-A- 5 316 547
- US-A1- 2008 300 523
- US-A1- 2011 288 611

## Beschreibung

Die vorliegende Erfindung betrifft eine Patellarsehnenbandage, die ein Bandagenelement mit einer Innenseite und einer Außenseite umfasst, wobei an der vorderen Innenseite sich zwei erste Pelottenkörper zum Aufbau eines infrapatellaren Drucks und zwei zweite Pelottenkörper, die seitlich der zwei ersten Pelottenkörper positioniert sind, befinden.

Patellarsehnenbandagen sind an sich bekannt. Eine typische Patellarsehnenbandage, auch Kniespange genannt, ist beispielsweise die "Kasseler Patellarsehnenbandage" wie sie in der DE 32 10 060 A1 beschrieben ist. Solche Patellarsehnenbandagen führen beim Anlegen der Bandage direkt unterhalb der Kniescheibe zu einer Druckbeaufschlagung der unter der Kniescheibe entspringenden Sehne und damit zu deren Vorspannung, wodurch Patellaschmerzen verringert werden können. Solche herkömmlichen Patellarsehnenbandagen weisen oftmals auf der Innenseite zwei hervorragende Pelottenköpfe auf, die über einen schmaleren, verjüngten Steg miteinander verbunden sind, sodass dieser Steg Platz für die Patellasehne lässt und die Patellasehne somit genau zwischen den beiden Pelottenköpfen liegen kann. Die herkömmlichen Patellarsehnenbandagen sind auf diese Wirkweise beschränkt.

Die US 2008/300523 A1 offenbart eine Patellarsehnenbandage, bei der zwei weitere Pelottenkörper auf der Rückseite des Knies positioniert sind.

Das der vorliegenden Erfindung zugrundeliegende technische Problem ist die Bereitstellung von verbesserten Patellarsehnenbandagen, insbesondere solchen, die weitere Funktionen, insbesondere synergistische Funktionen, beim Tragen ausüben. Insbesondere liegt der vorliegenden Erfindung das technische Problem zugrunde eine Patellarsehnenbandage bereitzustellen, die nicht nur zu einer Vorspannung der Patellasehne führt, sondern auch auf weitere Bereiche unterhalb der Kniescheibe wirkt, sodass eine Stabilisierung und/oder eine weitere Schmerzlinderung auftritt.

Die vorliegende Erfindung löst das ihr zugrundeliegende technische Problem durch die Gegenstände der unabhängigen Ansprüche.

Insbesondere löst die vorliegende Erfindung das technische Problem durch eine Patellarsehnenbandage gemäß Anspruch 1, umfassend ein Bandagenelement mit einer Innenseite und einer Außenseite und zwei an der vorderen Innenseite des Bandagenelements befindliche erste Pelottenkörper zum Aufbau eines infrapatellaren Drucks, wobei die Patellarsehnenbandage zusätzlich auf der Innenseite des Bandagenelements mindestens zwei zweite Pelottenkörper aufweist, wobei die zwei zweiten Pelottenkörper seitlich der zwei ersten Pelottenkörper positioniert sind.

Die vorliegende Erfindung sieht also vor, dass neben den zwei herkömmlichen Pelottenkörpern, also den zwei ersten Pelottenkörpern, die beispielsweise als über einen Steg miteinander verbundene Pelottenköpfe ausgebildet sind, zwei weitere Pelottenkörper vorgesehen sind, also insgesamt mindestens vier Pelottenkörper vorgesehen sind, wobei die vier Pelottenkörper bevorzugt nebeneinander, insbesondere bevorzugt annähernd in einer Reihe angeordnet sind. Die Anordnung der vier Pelottenkörper ist somit bevorzugt annähernd waagerecht, bezogen auf eine um ein Bein angelegte Patellarsehnenbandage.

Erfindungsgemäß sind die zwei zweiten Pelottenkörper seitlich von den zwei ersten Pelottenkörpern auf der Innenseite des Bandagenelements positioniert. Dabei bezieht sich "seitlich" im Zusammenhang mit der vorliegenden Erfindung auf eine Patellarsehnenbandage in einer Ausrichtung, wie sie vorliegt, wenn die Patellarsehnenbandage um ein Bein herum angebracht ist. Wenn die erfindungsgemäße Patellarsehnenbandage unterhalb der Patella angelegt wird, so sind die zwei ersten Pelottenkörper, wie aus dem Stand der Technik bekannt, links und rechts der Patellamitte unterhalb der Patella positioniert und üben dort einen Druck auf das Gewebe aus. Die zwei zweiten Pelottenkörper, die erfindungsgemäß seitlich der zwei ersten Pelottenkörper positioniert sind, sind somit bei einer angelegten Patellarsehnenbandage ebenfalls unterhalb der Patella positioniert, jedoch etwas weiter links beziehungsweise etwas weiter rechts von der Patellamitte als die ersten zwei Pelottenkörper.

Erfindungsgemäß sind die zwei zweiten Pelottenkörper so auf der Innenseite des Bandagenelements positioniert, dass sie im angelegten Zustand der Patellarsehnenbandage im seitlichen Bereich des Kniegelenks positioniert sind und auf dort verlaufende Muskel- und Sehnengruppen wirken.

Es wurde überraschenderweise gefunden, dass die zusätzlichen zwei zweiten Pelottenkörper im seitlichen Bereich des Kniegelenks die dort verlaufenden Muskel- und Sehnengruppen ansprechen und stimulieren können. Es hat sich herausgestellt, dass beispielsweise die Sehnen der Muskeln des "Pes anserinus" die Schlussrotation, Beugung und Streckung des Kniegelenks unterstützen. Dabei kommen diesen Muskeln bei jedem auszuführenden Schritt zum Einsatz, da sie die Verriegelung und die Öffnung der biomechanischen Kette des Bewegungsablaufs unterstützen. Durch den durch die zwei zweiten Pelottenkörper ausgeübten sensormotorischen Reiz kann das Kniegelenk schneller zwischen Verriegelung und Entriegelung umschalten. Zur Vorspannung der Patellasehne, die die Patellarsehnenbandagen aus dem Stand der Technik bewirken, kommt also zusätzlich eine weitere führungs- und sensormotorische Reizfunktion hinzu. Somit führen die zwei zweiten Pelottenkörper in synergistischer Weise mit den zwei ersten Pelottenkörpern zu einer höheren Kniegelenksstabilität und Gangsicherheit, wobei zugleich die Funktionalität der Kniegelenksbewegung verbessert wird.

Auch führen die zwei zweiten Pelottenkörper in einer Wechselwirkung mit dem darüber liegenden Bandagenelement, das beispielsweise als Gestrick ausgeführt sein kann, zu einer weiteren Stimulierung propriozeptiver Reize. Hierbei wird bei der Bewegung des Knies zwischen den Pelottenkörpern, dem darüber liegenden Bandagenelement und dem infrapatellaren Gewebe eine Spannung und Entspannung und somit eine zusätzliche Massagewirkung erreicht.

In einer bevorzugten Ausführungsform sind die zwei ersten Pelottenkörper und die zwei zweiten Pelottenkörper über Stege miteinander verbunden und bilden eine einstückige Pelotte. Bevorzugt sind die vier Pelottenkörper zumindest nahezu auf einer Linie angeordnet.

Bevorzugt sind die Stege zwischen den Pelottenkörpern schmaler und niedriger als die Pelottenkörper. Die einstückige Pelotte ist dabei bevorzugt aus einem einzigen Material gefertigt.

In einer bevorzugten Ausführungsform sind die zwei ersten Pelottenkörper und/oder die zwei zweiten Pelottenkörper in ihrer Position an dem Bandagenelement verstellbar.

In einer bevorzugten Ausführungsform sind die zwei ersten Pelottenkörper und/oder die zwei zweiten Pelottenkörper auswechselbar.

In einer Ausführungsform, bei der die zwei ersten Pelottenkörper und die zwei zweiten Pelottenkörper über Stege miteinander verbunden sind, ist somit bevorzugt die Pelotte in ihrer Position an dem Bandagenelement verstellbar und/oder auswechselbar.

In einer Ausführungsform, in der die einzelnen Pelottenkörper nicht über Stege miteinander verbunden sind, kann vorgesehen sein, dass jeweils die einzelnen Pelottenkörper in ihrer Position an dem Bandagenelement verstellbar und/oder auswechselbar sind. Bevorzugt sind dabei insbesondere die zwei ersten Pelottenkörper an dem Bandagenelement verstellbar beziehungsweise auswechselbar. Bevorzugt sind dabei insbesondere die zwei ersten Pelottenkörper an dem Bandagenelement in der Höhe verstellbar.

Die Erfindung sieht in einer besonderen Ausgestaltung somit vor, dass die Höhe und/oder die Nachgiebigkeit der zwei ersten Pelottenkörper und/oder der zwei zweiten Pelottenkörper oder der einstückigen Pelotte individuell gestaltbar und verstellbar ist, um die Druckbeaufschlagung auf den infrapatellaren Bereich individuell zu steuern. Dies kann im Rahmen eines Therapieplans oder zur Verwirklichung verschiedener prophylaktischer oder therapeutischer Ziele wünschenswert sein. Die Erfindung sieht in einer besonderen Ausgestaltung somit insbesondere vor, dass die Höhe der zwei ersten Pelottenkörper und/oder der zwei zweiten Pelottenkörper oder der einstückigen Pelotte individuell verstellbar ist. Die Erfindung sieht dazu beispielsweise vor, dass die erfindungsgemäßen Pelottenkörper oder die einstückige Pelotte lösbar mit dem Bandagenelement verbunden sind, und aus dieser zum Zwecke des Einstellens oder Anpassens der Höhe oder der Materialeigenschaften, besonders der Nachgiebigkeit, das heißt Shore-Härte, die Pelottenkörper oder die einstückige Pelotte herausnehmbar und anschließend wieder einsetzbar sind. Bei einer einfachen Höhenverstellung ist kein Austausch der Pelottenkörper oder der Pelotte nötig. Dabei ist bevorzugt vorgesehen, dass die zwei ersten Pelottenkörper und/oder die zwei zweiten Pelottenkörper zur Einstellung der Wirkung der Pelottenkörper auf den infrapatellaren Bereich auswechselbar sind, vor allem dass Pelottenkörper mit anderen Höhen, Größen oder anderen mechanischen Eigenschaften einsetzbar sind. Dies kann natürlich auch durch die Auswechselbarkeit der einstückigen Pelotte erreicht werden.

Zur Verstellbarkeit und/oder Austauschbarkeit können die Pelottenkörper oder die Pelotte beispielsweise über eine Klettverbindung mit der Innenseite des Bandagenelements verbunden sein.

Die zwei ersten Pelottenkörper und/oder die zwei zweiten Pelottenkörper können jegliche geeignete und dem Fachmann bekannte Form aufweisen. Bevorzugt handelt es sich bei den Pelottenkörpern um Pelottenköpfe. Bevorzugt handelt es sich bei den ersten zwei Pelottenkörpern um Pelottenköpfe. Bevorzugt handelt es sich bei den zweiten zwei Pelottenkörpern um Pelotten köpfe.

Die Pelottenkörper können beispielsweise einen runden oder ovalen Querschnitt haben. Insbesondere können die Pelottenkörper eine annähernd halbkugelförmige, beispielsweise halbkugelförmige oder halbeiförmige, Form haben. Jedoch sind natürlich auch andere Formen denkbar, beispielsweise können die Pelottenkörper pyramidenförmig, würfelförmig, kastenförmig, polyederförimg, torusförmig, hohlzylinderförmig, kreiszylinderförmig, kegelförmig oder prismenförmig sein.

Die zwei ersten Pelottenkörper und/oder die zwei zweiten Pelottenkörper oder die einstückige Pelotte sind bevorzugt aus einem dauerelastischen Werkstoff, insbesondere Silikonkautschuk oder Polyurethan, gebildet. Die Erfindung ist jedoch nicht auf diese Werkstoffe beschränkt. Der Fachmann kennt gleichermaßen geeignete Werkstoffe. Dabei ist insbesondere das physikalisch-mechanische Verhalten des Werkstoffs, vor allem das Elastizitätsmodul, an das Weichteilgewebe des Kniegelenks angepasst.

In einer bevorzugten Ausführungsform weisen die zwei ersten Pelottenkörper und/oder die zwei zweiten Pelottenkörper Erhebungen oder Vertiefungen auf. Bevorzugt handelt es sich um Erhebungen. Bevorzugt sind die Erhebungen noppenförmig. Bevorzugt weisen die Pelottenkörper also mehrere Noppen auf. Bevorzugt weist ein Pelottenkörper zwischen 2 und 20 Noppen auf. Bevorzugt bestehen die Noppen aus dem gleichen Material wie der Pelottenkörper.

Bevorzugt ist das Bandagenelement streifenförmig beziehungsweise als Band oder als Gurt ausgestaltet. Bevorzugt ist das Bandagenelement überwiegend als Gestrick und/oder Gewirk ausgestaltet.

Bevorzugt ist das Bandagenelement ein Band oder ein Gurt, wobei das Band oder der Gurt um ein Bein herumgelegt werden kann und dann mittels mindestens eines Verschlusselements ringförmig geschlossen werden kann. Bevorzugt sind zwei Verschlusselemente vorgesehen. Bevorzugt sind die Verschlusselemente so ausgestaltet, dass sie ein Festziehen der Patellarsehnenbandage an dem Bein erlauben.

Ein bevorzugtes Verschlusselement weist eine Umlenköse und einen Klettverschluss auf. Dabei kann der Klettverschluss durch die Umlenköse zum Verschließen durchgeführt werden und dadurch das Bandagenelement am Bein festgezogen werden und diese Position durch Verkletten des Klettverschlusses mit einem weiteren Teil des Bandagenelements fixiert werden.

In einer bevorzugten Ausführungsform weist das Bandagenelement ein vorderes Funktionselement und ein Fixierungselement, insbesondere einen Fixierungsgurt, auf. In einer bevorzugten Ausführungsform ist das Fixierungselement an mindestens einem Endbereich des Funktionselements über ein Befestigungselement längenverstellbar befestigt.

Das Funktionselement dient in dieser Ausführungsform als Träger der Pelottenkörper oder der Pelotte. Das Fixierungselement dient dem Festziehen des Funktionselements am Bein und dem dadurch bewirkten Anpressen der Pelottenkörper auf den infrapatellaren Bereich.

Bevorzug besteht das Funktionselement aus einem Gestrick. Bevorzugt wird das Fixierungselement aus einem Gestrick oder einem Gewirk oder einer anderen geeigneten Materialkomponente gebildet. Besonders bevorzugt wird das Fixierungselement aus einem Gewirk gebildet.

In einer bevorzugten Ausführungsform ist das mindestens eine Befestigungselement auf der Außenseite des Funktionselements am Funktionselement befestigt.

In einer bevorzugten Ausführungsform ist das mindestens eine Befestigungselement am Funktionselement im Bereich eines der zwei zweiten Pelottenkörper befestigt.

In einer bevorzugten Ausführungsform ist das Fixierungselement an beiden Endbereichen des Funktionselements über jeweils ein Befestigungselement längenverstellbar befestigt. Bevorzugt sind die zwei Befestigungselemente auf der der Außenseite des Funktionselements am Funktionselement befestigt, wobei jeweils ein Befestigungselement im Bereich jeweils eines der zwei zweiten Pelottenkörper befestigt ist.

In einer bevorzugten Ausführungsform weist das Fixierungselement mindestens einen Klettverschluss auf. In einer bevorzugten Ausführungsform ist das mindestens eine Befestigungselement eine Umlenköse.

In einer bevorzugten Ausführungsform weist das Fixierungselement an den Enden zwei Klettverschlüsse auf. In einer bevorzugten Ausführungsform weist das Funktionselement, insbesondere an seiner Außenseite, zwei Umlenkösen auf. In einer solchen Ausführungsform erfolgt also die Befestigung des Fixierungselements am Funktionselement durch zwei Klettverschlüsse, die jeweils durch eine der beiden Umlenkösen hindurchgezogen werden können und dann an dem Fixierungselement festgeklettet werden können. Dadurch ist auf einfache Weise eine gewünschte Längenanpassung der Patellarsehnenbandage möglich.

In einer bevorzugten Ausführungsform weist also die Patellarsehnenbandagen ein vorderes Funktionselement und ein Fixierungselement auf, wobei das Fixierungselement an den beiden Endbereichen des Funktionselements über je ein Befestigungselement längenverstellbar befestigt ist und wobei das erste Befestigungselement im Bereich des einen zweiten Pelottenkörpers positioniert ist und das zweite Befestigungselement im Bereich des anderen zweiten Pelottenkörpers positioniert ist. Bevorzugt sind die beiden Befestigungselemente dabei auf der Außenseite des Bandagenelements, insbesondere des Funktionselements positioniert.

Diese bevorzugte Ausführungsform führt zu einem zusätzlichen technischen Vorteil, nämlich dass die zwei Befestigungselemente als Druckeinleitungspunkte des Fixierungsgurts direkt auf die zwei zweiten Pelotten dienen können. Durch die bevorzugte Positionierung wird die durch das Fixierungselement übertragene Spannung genau an der Position auf das Funktionselement übertragen, an der die zwei zweiten Pelotten liegen. Dadurch wird in vorteilhafter Weise erreicht, dass die zwei zweiten Pelottenkörper den Druck, der von dem Fixierungselement auf die zwei Befestigungselemente übertragen wird, von den Befestigungselementen abfangen. Dadurch kann eine besonders gute Druckwirkung der zwei zweiten Pelottenkörper auf den infrapatellaren Bereich erreicht werden.

Die vorliegende Erfindung betrifft auch eine Patellarsehnenpelotte gemäß Anspruch 11 umfassend vier nahezu auf einer Linie angeordnete Pelottenkörper, die über Stege miteinander verbunden sind.

Bevorzugte Ausführungsformen der erfindungsgemäßen Patellasehenpelotte ergeben sich aus den oben dargelegten Ausführungsformen der Pelottenkörper beziehungsweise der Pelotte.

Eine erfindungsgemäße Patellarsehnenpelotte eignet sich insbesondere als Element zum Aufrüsten einer Patellarsehnenbandage.

Gegenstand der Erfindung ist auch eine Patellarsehnenbandage, welche die erfindungsgemäße Patellarsehnenpelotte enthält. Diese kann besonders als Gestrickorthese mit eingelegter erfindungsgemäßen Patellarsehnenpelotte ausgestaltet sein.

Die vorliegende Erfindung betrifft auch eine erfindungsgemäße Patellarsehnenbandage oder eine erfindungsgemäße Patellarsehnenpelotte zur Schmerzlinderung, insbesondere bei einem vorderen Knieschmerz. Ein solcher vorderer Knieschmerz kann beispielsweise durch ein Patellaspitzensyndrom, durch eine Chondropathia patellae, durch eine femoropatellare Arthrose, durch eine Kreuzbandinsuffizienz des vorderen Kreuzbands, durch eine Quadrizepsparese oder durch eine Muskeldistrophie verursacht werden. Auch eine Verwendung bei einem postoperativen oder posttraumatischen Rehatraining ist möglich. Die Erfindung betrifft entsprechend eben solche Verwendungen der erfindungsgemäßen Patellarsehnenbandage oder der erfindungsgemäßen Patellarsehnenpelotte.

Offenbart ist auch die prophylaktische und/oder therapeutische Verwendung der erfindungsgemäßen Patellarsehnenbandage oder der erfindungsgemäßen Patellarsehnenpelotte zur Behandlung des vorderen Knieschmerzsyndroms.

Die Erfindung betrifft ebenfalls eine erfindungsgemäße Patellarsehnenbandage oder eine erfindungsgemäße Patellarsehnenpelotte für eine entsprechende prophylaktische und/oder therapeutische Verwendung.

Die Erfindung wird anhand der nachfolgenden Figuren näher beschrieben, ohne dass die dort dargestellten Ausführungsformen der Erfindung beschränkend zu verstehen sind.

Es zeigen:
Figur 1 eine Seitenansicht einer bevorzugten Ausführungsform der erfindungsgemäßen Patellarsehnenbandage;
Figur 2 eine Seitenansicht einer weiteren bevorzugten Ausführungsform der erfindungsgemäßen Patellarsehnenbandage;
Figur 3 eine Schrägansicht der Patellarsehnenbandage aus Figur 1;
Figur 4 eine Seitenansicht einer weiteren bevorzugten Ausführungsform der erfindungsgemäßen Patellarsehnenbandage;
Figur 5 zwei verschiedene Ausführungsformen der erfindungsgemäßen Patellarsehnenpelotte.

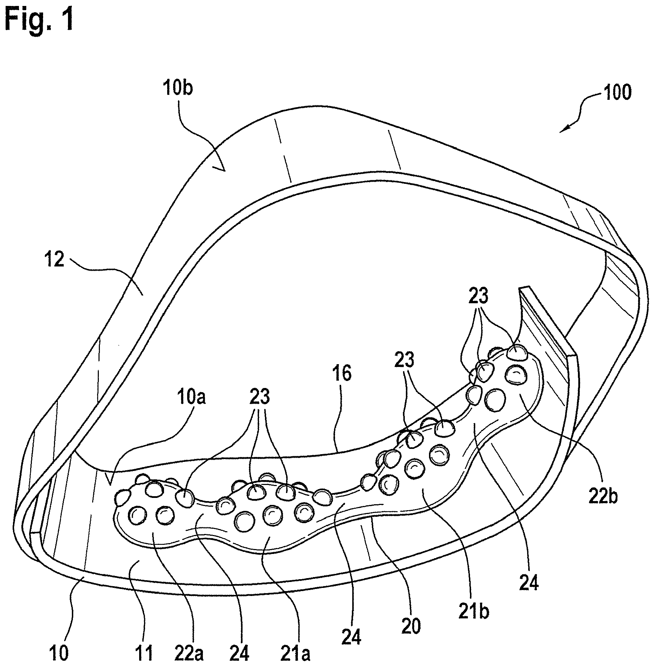

Figur 1 zeigt eine bevorzugte Ausführungsform der erfindungsgemäßen Patellarsehnenbandage (100) in Seitenansicht. Die Patellarsehnenbandage (100) umfasst ein Bandagenelement (10) mit einem Funktionselement (11) und einem Fixierungselement in Form eines Fixierungsgurtes (12) auf. Das Bandagenelement (10) hat eine Innenseite (10a) und eine Außenseite (10b). Das Funktionselement (11) bildet die Vorderseite des Bandagenelements (10) und wird unterhalb der Patella getragen. Im oberen Bereich des Funktionselements (11) befindet sich eine Ausnehmung (16) für die Patella. Das Funktionselement (11) kann beispielsweise aus einem Gestrick gebildet sein, das Fixierungselement (12) kann aus einem Gewirk oder Gestrick gebildet sein.

Auf der Innenseite (10a) des Funktionselements (11) ist eine Pelotte (20) positioniert. Diese weist zwei erste Pelottenkörper in Form von Pelottenköpfen (21a, 21b) mit einem ovalen Querschnitt und zwei zweite Pelottenkörper in Form von Pelottenköpfen (22a, 22b) mit einem annähernd runden Querschnitt auf. Die zwei zweiten Pelottenköpfe (22a, 22b) sind seitlich der zwei ersten Pelottenköpfe (21a, 21b) positioniert. Die insgesamt vier Pelottenköpfe (22a, 21a, 21b, 22b) sind über Stege (24) miteinander verbunden und bilden so die einstückige Pelotte (20). Wenn die erfindungsgemäße Patellarsehnenbandage unterhalb der Patella angelegt wird, so sind die zwei ersten Pelottenkörper, wie aus dem Stand der Technik bekannt, links und rechts der Patellamitte unterhalb der Patella positioniert und üben dort einen Druck auf das Gewebe aus. Die zwei zweiten Pelottenkörper, die erfindungsgemäß seitlich der zwei ersten Pelottenkörper positioniert sind, sind somit bei einer angelegten Patellarsehnenbandage ebenfalls unterhalb der Patella positioniert, jedoch etwas weiter links beziehungsweise etwas weiter rechts von der Patellamitte als die ersten zwei Pelottenkörper. Durch den erfindungsgemäßen Aufbau der Pelottenkörper wird der in der Beschreibung dargelegte vorteilhafte Effekt beim Tragen der Patellarsehnenbandage (100) unterhalb der Kniescheibe erreicht.

Die vier Pelottenköpfe (22a, 21a, 21b, 22b) weisen jeweils mehrere Erhebungen in Form von Noppen (23) auf. Dies führt zu einer zusätzlichen vorteilhaften Massage und Reizübertragung durch alle vier Pelottenköpfe (22a, 21a, 21b, 22b).

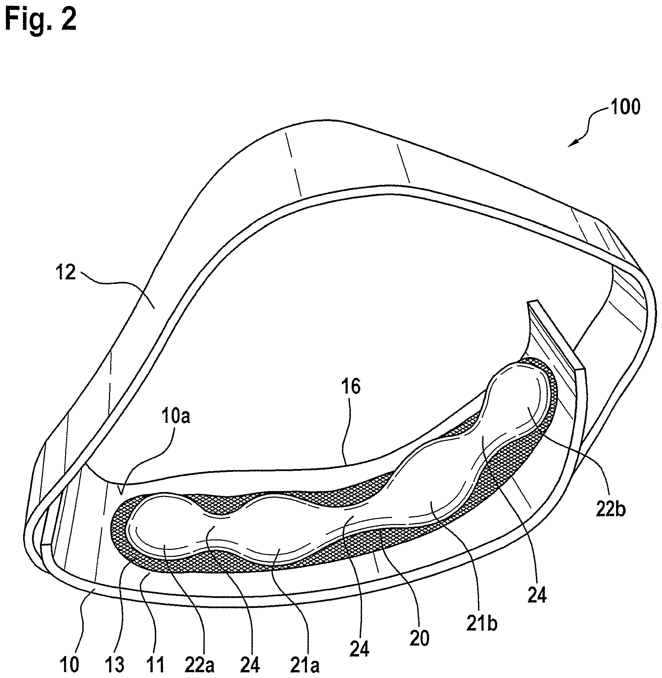

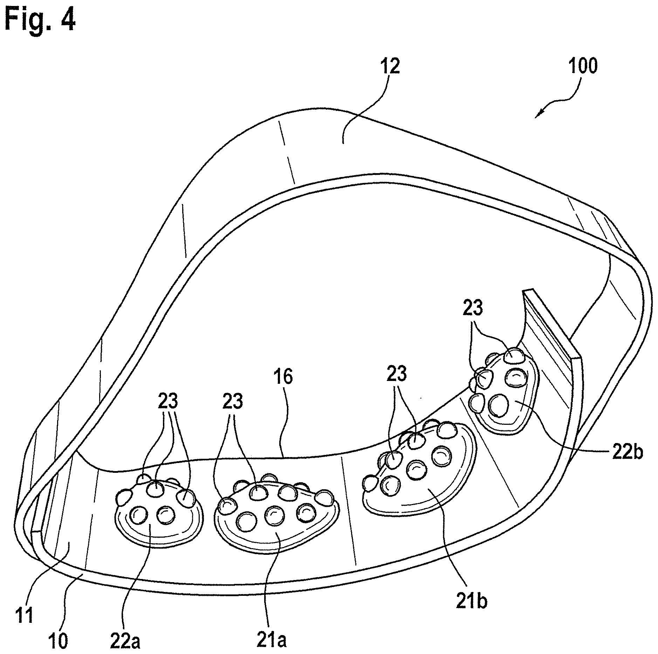

Weitere Ausgestaltungsmöglichkeiten kann der Fachmann auch den Figuren 2 und 4 entnehmen.

Figur 2 zeigt eine alternative Ausführungsform der erfindungsgemäßen Patellarsehnenbandage (100) in Seitenansicht. Die Patellarsehnenbandage (100) umfasst wieder wie in Figur 1 das Bandagenelement (10) mit einem Funktionselement (11) mit der Ausnehmung (16) und einem Fixierungselement in Form eines Fixierungsgurtes (12) auf.

Auf der Innenseite (10a) des Funktionselements (11) ist eine Pelotte (20) positioniert. Diese weist wieder zwei erste Pelottenkörper in Form von Pelottenköpfen (21a, 21b) mit einem ovalen Querschnitt und zwei zweite Pelottenkörper in Form von Pelottenköpfen (22a, 22b) mit einem annähernd runden Querschnitt auf. Die zwei zweiten Pelottenköpfe (22a, 22b) sind seitlich der zwei ersten Pelottenköpfe (21a, 21b) positioniert. Die insgesamt vier Pelottenköpfe (22a, 21a, 21b, 22b) sind über Stege (24) miteinander verbunden und bilden so die einstückige Pelotte (20).

Die Pelotte (20) ist über eine Klettverbindung (13) mit dem Funktionselement (11) reversibel verbunden und kann somit in ihrer Position, beispielsweise in der Höhe, in Bezug auf das Funktionselement (11) verstellt werden oder durch eine andere Pelotte ausgetauscht werden.

Weitere Ausgestaltungsmöglichkeiten kann der Fachmann auch den Figuren 1 und 3 bis 4 entnehmen.

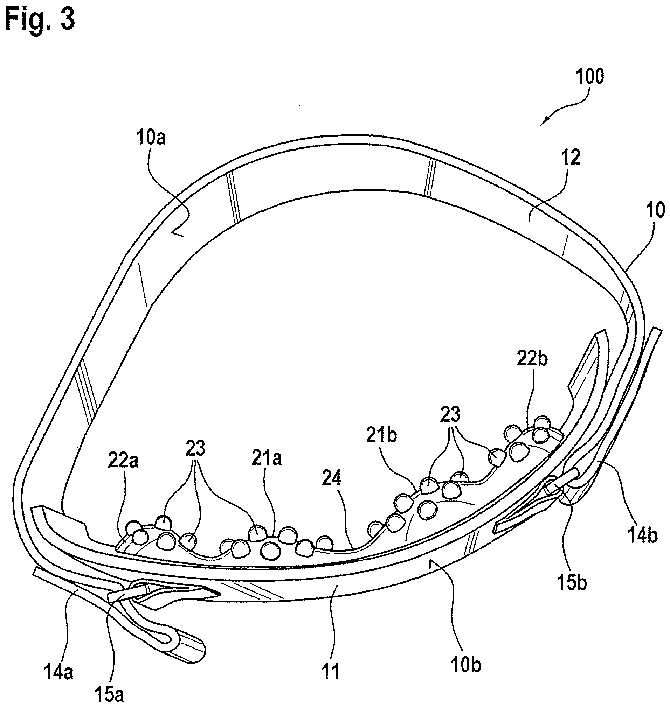

Figur 3 zeigt die Patellarsehnenbandage (100) von Figur 1 in Schrägansicht. Es sind wieder das Bandagenelement (10) mit Innenseite (10a) und Außenseite (10b) mit dem Funktionselement (11) und dem Fixierungselement in Form eines Fixierungsgurtes (12) zu sehen. Auch die Pelotte (20) mit den über Stege (24) miteinander verbundenen vier Pelottenköpfen (22a, 21a, 21b, 22b) und den Noppen (23) ist gezeigt.

Der Fixierungsgurt (12) ist an seinen beiden Enden jeweils mit einem Endbereich des Funktionselements (11) reversibel verbunden um die ringförmige Bandage (100) zu bilden. Die zwei reversiblen und längenverstellbaren Verbindungen zwischen Fixierungsgurt (12) und Funktionselement werden durch zwei Klettverbindungen (14a, 14b) am Fixierungsgurt (12) und zwei Umlenkösen (15a, 15b) am Funktionselement (11), durch die der Fixierungsgurt (12) im Bereich der Klettverbindungen (14a, 14b) hindurchgezogen sind, ermöglicht. Somit ist ein einfaches Anziehen und Festziehen der Patellarsehnenbandage (100) an einem Bein möglich.

Die beiden Umlenkösen (15a, 15b) sind auf der Außenseite (10b) des Funktionselements (11) positioniert und zwar genau dort wo auf der Innenseite (10a) des Funktionselements (11) die zwei zweiten Pelottenköpfe (22a, 22b) positioniert sind. Somit werden die durch den Fixierungsgurt (12) erzeugten Kräfte in vorteilhafter Weise über die erste Umlenköse (15a) direkt auf den ersten zweiten Pelottenkopf (22a) und über die zweite Umlenköse (15b) direkt auf den zweiten zweiten Pelottenkopf (22b) übertragen. Dadurch wird in vorteilhafter Weise erreicht, dass die zwei zweiten Pelottenköpfe (22a, 22b) diese Kraft direkt von den Umlenkösen (15a, 15b) abfangen und als Druck aufs Bein übertragen. Dadurch kann eine besonders gute Druckwirkung der zwei zweiten Pelottenköpfe (22a, 22b) auf den infrapatellaren Bereich erreicht werden.

Weitere Ausgestaltungsmöglichkeiten kann der Fachmann auch den Figuren 2 und 4 entnehmen.

Figur 4 zeigt eine weitere Ausführungsform der erfindungsgemäßen Patellarsehnenbandage (100) in Seitenansicht. Die Patellarsehnenbandage (100) umfasst wieder wie in Figur 1 das Bandagenelement (10) mit einem Funktionselement (11) mit der Ausnehmung (16) und einem Fixierungselement in Form eines Fixierungsgurtes (12) auf.

Auf der Innenseite (10a) des Funktionselements (11) sind zwei erste Pelottenkörper in Form von Pelottenköpfen (21a, 21b) mit einem ovalen Querschnitt und zwei zweite Pelottenkörper in Form von Pelottenköpfen (22a, 22b) mit einem annähernd runden Querschnitt positioniert. Die zwei zweiten Pelottenköpfe (22a, 22b) sind wieder seitlich der zwei ersten Pelottenköpfe (21a, 21b) positioniert. Die insgesamt vier Pelottenköpfe (22a, 21a, 21b, 22b) sind hier nicht über Stege miteinander verbunden und bilden somit keine einstückige Pelotte. Die vier Pelottenköpfe (22a, 21a, 21b, 22b) weisen jeweils mehrere Erhebungen in Form von Noppen (23) auf.

Die vier Pelottenköpfe (22a, 21a, 21b, 22b) können beispielsweise über eine Klettverbindung mit dem Funktionselement (11) reversibel verbunden sein und kann somit in ihrer Position, beispielsweise in der Höhe, in Bezug auf das Funktionselement (11) verstellt werden oder durch eine andere Pelotte ausgetauscht werden. Alternativ kann aber auch vorgesehen sein, dass die vier Pelottenköpfe (22a, 21a, 21b, 22b) fest mit dem Funktionselement (11) verbunden sind.

Weitere Ausgestaltungsmöglichkeiten kann der Fachmann auch den Figuren 1 bis 3 entnehmen.

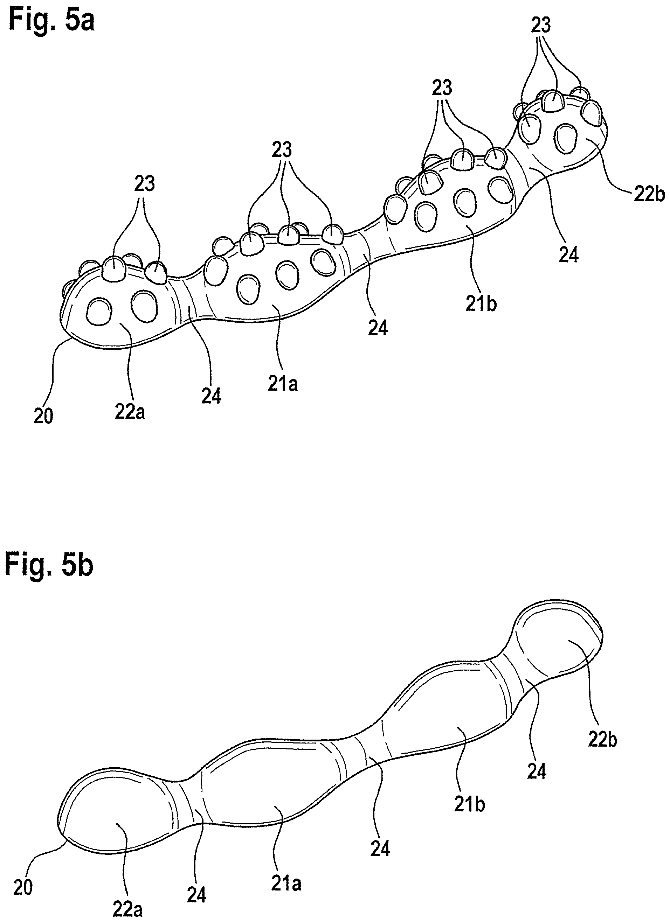

Figur 5 zwei verschiedene Ausführungsformen der erfindungsgemäßen Patellarsehnenpelotte (20).

Die Patellarsehnenpelotte (20) in Figur 5a weist zwei erste Pelottenkörper in Form von Pelottenköpfen (21a, 21b) mit einem ovalen Querschnitt und zwei zweite Pelottenkörper in Form von Pelottenköpfen (22a, 22b) mit einem annähernd runden Querschnitt auf. Die zwei zweiten Pelottenköpfe (22a, 22b) sind seitlich der zwei ersten Pelottenköpfe (21a, 21b) positioniert. Die insgesamt vier Pelottenköpfe (22a, 21a, 21b, 22b) sind über in der Mitte verjüngte Stege (24) miteinander verbunden und bilden so die einstückige Pelotte (20). Die vier Pelottenköpfe (22a, 21a, 21b, 22b) weisen jeweils mehrere Erhebungen in Form von Noppen (23) auf.

Die Patellarsehnenpelotte (20) in Figur 5b weist wie die Pelotte aus Figur 5a zwei erste Pelottenkörper in Form von Pelottenköpfen (21a, 21b) und zwei zweite Pelottenkörper in Form von Pelottenköpfen (22a, 22b) auf. Die insgesamt vier Pelottenköpfe (22a, 21a, 21b, 22b) sind über in der Mitte verjüngte Stege (24) miteinander verbunden und bilden so die einstückige Pelotte (20).

Die Patellarsehnenpelotten (20) aus den Figuren 5a und 5b eignen sich beispielsweise als Element zum Aufrüsten einer Patellarsehnenbandage. Diese kann zum Beispiel als Gestrickorthese mit eingelegter Patellarsehnenpelotte (20) ausgestaltet sein.

## Patentansprüche

1. Patellarsehnenbandage (100), umfassend ein Bandagenelement (10) mit einer Innenseite (10a) und einer Außenseite (10b) und zwei an der vorderen Innenseite (10a) des Bandagenelements befindliche erste Pelottenkörper (21a, 21b) zum Aufbau eines infrapatellaren Drucks, wobei die Patellarsehnenbandage (100) zusätzlich auf der Innenseite (10a) des Bandagenelements (100) mindestens zwei zweite Pelottenkörper (22a, 22b) aufweist, wobei die zwei zweiten Pelottenkörper (22a, 22b) seitlich der zwei ersten Pelottenkörper (21a ,21b) positioniert sind, **dadurch gekennzeichnet, dass** die zwei zweiten Pelottenkörper (22a, 22b) so auf der Innenseite (10a) des Bandagenelements (10) positioniert sind, dass sie im angelegten Zustand der Patellarsehnenbandage (100) im seitlichen Bereich des Kniegelenks positioniert sind und auf dort verlaufende Muskel- und Sehnengruppen wirken.

2. Patellarsehnenbandage nach Anspruch 1, wobei die zwei ersten Pelottenkörper (21,a 21b) und die zwei zweiten Pelottenkörper (22a, 22b) über Stege (24) miteinander verbunden sind und eine einstückige Pelotte (20) bilden und wobei die vier Pelottenkörper (21a, 21b, 22a, 22b) zumindest nahezu auf einer Linie angeordnet sind.

3. Patellarsehnenbandage nach einem der vorstehenden Ansprüche, wobei die zwei ersten Pelottenkörper (21a, 21b) und/oder die zwei zweiten Pelottenkörper (22a, 22b) in ihrer Position an dem Bandagenelement (10) verstellbar sind.

4. Patellarsehnenbandage nach einem der vorstehenden Ansprüche, wobei die zwei ersten Pelottenkörper (21a, 21b) und/oder die zwei zweiten Pelottenkörper (22a, 22b) höhenverstellbar und/oder auswechselbar sind.

5. Patellarsehnenbandage nach einem der vorstehenden Ansprüche, wobei die zwei ersten Pelottenkörper (21a, 21b) und/oder die zwei zweiten Pelottenkörper (22a, 22b) Erhebungen (23) oder Vertiefungen aufweisen.

6. Patellarsehnenbandage nach einem der vorstehenden Ansprüche, wobei das Bandagenelement (10) ein vorderes Funktionselement (11) und ein Fixierungselement (12), insbesondere Fixierungsgurt (12), aufweist, wobei das Fixierungselement (12) an mindestens einem Endbereich des Funktionselements (11) über ein Befestigungselement (14a, 15a, 14b, 15b) längenverstellbar befestigt ist.

7. Patellarsehnenbandage nach Anspruch 6, wobei das mindestens eine Befestigungselement (14a, 15a, 14b, 15b) auf der Außenseite (10b) des Funktionselements (11) am Funktionselement (11) befestigt ist.

8. Patellarsehnenbandage nach Anspruch 6 oder Anspruch 7, wobei das mindestens eine Befestigungselement (14a, 15a, 14b, 15b) am Funktionselement (11) im Bereich eines der zwei zweiten Pelottenkörper (22a, 22b) befestigt ist.

9. Patellarsehnenbandage nach Anspruch 6, wobei das Fixierungselement (12) an beiden Endbereichen des Funktionselements (11) über jeweils ein Befestigungselement (14a, 15a, 14b, 15b) längenverstellbar befestigt ist, wobei die Befestigungselemente auf der der Außenseite (10b) des Funktionselements (11) am Funktionselement befestigt sind und wobei jeweils ein Befestigungselement (14a, 15a, 14b, 15b) im Bereich jeweils eines der zwei zweiten Pelottenkörper (22a, 22b) befestigt ist.

10. Patellarsehnenbandage nach einem der Ansprüche 6 bis 9, wobei das Fixierungselement (12) mindestens einen Klettverschluss (14a, 14b) aufweist und wobei das mindestens eine Befestigungselement eine Umlenköse (15a, 15b) ist.

11. Patellarsehnenpelotte (20) zum Aufrüsten einer Patellarsehnenbandage mit einem Bandagenelement (10), wobei die Patellarsehnenpelotte (20) vier nahezu auf einer Linie angeordnete Pelottenkörper (21a, 21b, 22a, 22b) umfasst, die über Stege (24) miteinander verbunden sind, **dadurch gekennzeichnet, dass** zwei erste Pelottenkörper (21a, 21b) der vier nahezu auf einer Linie angeordneten Pelottenkörper (21a, 21b, 22a, 22b) zum Aufbau eines infrapatellaren Drucks dienen und zwei zweite Pelottenkörper (22a, 22b) der vier nahezu auf einer Linie angeordneten Pelottenkörper (21a, 21b, 22a, 22b) so auf der Innenseite (10a) des Bandagenelements (10) positioniert sind, dass sie im angelegten Zustand der Patellarsehnenbandage (100) im seitlichen Bereich des Kniegelenks positioniert sind und auf dort verlaufende Muskel- und Sehnengruppen wirken.

## Claims

1. Patellar tendon bandage (100), comprising a bandage element (10) with an inner face (10a) and an outer face (10b) and two first pad bodies (21a, 21b) located on the front inner face (10a) of the bandage elements for building up an infrapatellar pressure, the patellar tendon bandage (100) additionally having at least two second pad bodies (22a, 22b) on the inner face (10a) of the bandage element (100), the two second pad bodies (22a, 22b) being positioned to the side of the two first pad bodies (21a, 21b), **characterised in that** the two second pad bodies (22a, 22b) are positioned on the inner face (10a) of the bandage element (10) in such a way that they are positioned in the side region of the knee joint, and act on muscle and tendon groups extending there, when the patellar tendon bandage (100) is applied.

2. Patellar tendon bandage according to claim 1, wherein the two first pad bodies (21a, 21b) and the two second pad bodies (22a, 22b) are interconnected via webs (24) and form a single-piece pad (20), and wherein the four pad bodies (21a, 21b, 22a, 22b) are arranged at least nearly on a line.

3. Patellar tendon bandage according to any of the preceding claims, the two first pad bodies (21a, 21b) and/or the two second pad bodies (22a, 22b) being adjustable in the position thereof on the bandage element (10).

4. Patellar tendon bandage according to any of the preceding claims, wherein the two first pad bodies (21a, 21b) and/or the two second pad bodies (22a, 22b) are height-adjustable and/or replaceable.

5. Patellar tendon bandage according to any of the preceding claims, wherein the two first pad bodies (21a, 21b) and/or the two second pad bodies (22a, 22b) have elevations (23) or depressions.

6. Patellar tendon bandage according to any of the preceding claims, wherein the bandage element (10) has a front functional element (11) and a fixing element (12), in particular a fixing belt (12), the fixing element (12) being fastened in a length-adjustable manner on at least one end region of the functional element (11) via a fastening element (14a, 15a, 14b, 15b).

7. Patellar tendon bandage according to claim 6, wherein the at least one fastening element (14a, 15a, 14b, 15b) is fastened on the functional element (11) on the outer face (10b) of the functional element (11).

8. Patellar tendon bandage according to claim 6 or claim 7, wherein the at least one fastening element (14a, 15a, 14b, 15b) is fastened on the functional element (11) in the region of one of the two second pad bodies (22a, 22b).

9. Patellar tendon bandage according to claim 6, wherein the fixing element (12) is fastened in a length-adjustable manner to the two end regions of the functional element (11) via a fastening element (14a, 15a, 14b, 15b) in each case, the fastening elements on the outer face (10b) of the functional element (11) being fastened on the functional element and each fastening element (14a, 15a, 14b, 15b) being fastened in the region of an associated one of the two second pad bodies (22a, 22b).

10. Patellar tendon bandage according to any of claims 6 to 9, wherein the fixing element (12) has at least one hook-and-loop fastener (14a, 14b) and wherein the at least one fastening element is a deflection eyelet (15a, 15b).

11. Patellar tendon pad (20) for upgrading a patellar tendon bandage with a bandage element (10), the patellar tendon pad (20) comprising four pad
bodies (21a, 21b, 22a, 22b) arranged nearly on a line and interconnected via webs (24), **characterised in that** two first pad bodies (21a, 21b) of the four pad
bodies (21a, 21b, 22a, 22b) arranged nearly on a line serve to build up an infrapatellar pressure and two second pad bodies (22a, 22b) of the four pad
bodies (21a, 21b, 22a, 22b) arranged nearly on a line are positioned on the inner face (10a) of the bandage element (10) in such a way that they are positioned in the side region of the knee joint, and act on muscle and tendon groups extending there, when the patellar tendon bandage (100) is applied.

## Revendications

1. Bandage pour tendon rotulien (100), comprenant un élément de bandage (10) muni d'un côté intérieur (10a) et d'un côté extérieur (10b) et de deux premiers corps de pelote (21a, 21b) se trouvant sur le côté intérieur avant (10a) de l'élément de bandage, destinés à établir une pression infra-rotulienne, le bandage pour tendon rotulien (100) présentant en plus sur le côté intérieur (10a) de l'élément de bandage (100) au moins deux deuxièmes corps de pelote (22a, 22b), les deux deuxièmes corps de pelote (22a, 22b) étant positionnés latéralement des deux premiers corps de pelote (21a, 21b), **caractérisé en ce que** les deux deuxièmes corps de pelote (22a, 22b) sont positionnés sur le côté intérieur (10a) de l'élément de bandage (10) de manière à ce qu'à l'état posé du bandage pour tendon rotulien (100), ils soient positionnés dans la partie latérale de l'articulation du genou et agissent sur des groupes de muscles et de tendons qui s'y étendent.

2. Bandage pour tendon rotulien selon la revendication 1, dans lequel les deux premiers corps de pelote (21a, 21b) et les deux deuxièmes corps de pelote (22a, 22b) sont reliés entre eux par des nervures (24) et forment une pelote (20) d'une seule pièce, et dans lequel les quatre corps de pelote (21a, 21b, 22a, 22b) sont disposés au moins presque sur une ligne.

3. Bandage pour tendon rotulien selon l'une quelconque des revendications précédentes, dans lequel les deux premiers corps de pelote (21a, 21b) et/ou les deux deuxièmes corps de pelote (22a, 22b) sont réglables dans leur position sur l'élément de bandage (10).

4. Bandage pour tendon rotulien selon l'une quelconque des revendications précédentes, dans lequel les deux premiers corps de pelote (21a, 21b) et/ou les deux deuxièmes corps de pelote (22a, 22b) sont réglables en hauteur et/ou sont interchangeables.

5. Bandage pour tendon rotulien selon l'une quelconque des revendications précédentes, dans lequel les deux premiers corps de pelote (21a, 21b) et/ou les deux deuxièmes corps de pelote (22a, 22b) présentent des élévations (23) ou des creux.

6. Bandage pour tendon rotulien selon l'une quelconque des revendications précédentes, dans lequel l'élément de bandage (10) présente un élément fonctionnel avant (11) et un élément de fixation (12), notamment une sangle de fixation (12), dans lequel l'élément de fixation (12) est fixé de manière réglable en longueur sur au moins une partie terminale de l'élément fonctionnel (11) par l'intermédiaire d'un élément de fixation (14a, 15a, 14b, 15b).

7. Bandage pour tendon rotulien selon la revendication 6, dans lequel l'au moins un élément de fixation (14a, 15a, 14b, 15b) est fixé sur l'élément fonctionnel (11), sur le côté extérieur (10b) de l'élément fonctionnel (11).

8. Bandage pour tendon rotulien selon la revendication 6 ou la revendication 7, dans lequel l'au moins un élément de fixation (14a, 15a, 14b, 15b) est fixé sur l'élément fonctionnel (11) au niveau de l'un des deux deuxièmes corps de pelote (22a, 22b).

9. Bandage pour tendon rotulien selon la revendication 6, dans lequel l'élément de fixation (12) est fixé de manière réglable en longueur sur les deux parties terminales de l'élément fonctionnel (11) par l'intermédiaire de respectivement un élément de fixation (14a, 15a, 14b, 15b), dans lequel les éléments de fixation sont fixés sur l'élément fonctionnel, sur le côté extérieur (10b) de l'élément fonctionnel (11), et dans lequel respectivement un élément de fixation (14a, 15a, 14b, 15b) est fixé à la zone de respectivement l'un des deux deuxièmes corps de pelote (22a, 22b).

10. Bandage pour tendon rotulien selon l'une quelconque des revendications 6 à 9, dans lequel l'élément de fixation (12) présente au moins une fermeture autoagrippante (14a, 14b) et dans lequel l'au moins un élément de fixation est un oeillet de renvoi (15a, 15b).

11. Pelote pour tendon rotulien (20) destinée à augmenter un bandage pour tendon rotulien muni d'un élément de bandage (10), la pelote pour tendon rotulien (20) comprenant quatre corps de pelote (21a, 21b, 22a, 22b) disposés presque sur une ligne, lesquels sont reliés entre eux par des nervures (24), **caractérisée en ce que** deux premiers corps de pelote (21a, 21b) des quatre corps de pelote (21a, 21b, 22a, 22b) disposés presque sur une ligne servent à établir une pression infra-rotulienne et **en ce que** deux deuxièmes corps de pelote (22a, 22b) des quatre corps de pelote (21a, 21b, 22a, 22b) disposés presque sur une ligne sont positionnés sur le côté intérieur (10a) de l'élément de bandage (10) de manière à ce qu'à l'état posé du bandage pour tendon rotulien (100), ils soient positionnés dans la partie latérale de l'articulation du genou et agissent sur des groupes de muscles et de tendons qui s'y étendent.
